# EUROPEAN PATENT SPECIFICATION

(11) **EP 1 953 554 B1**
(45) Date of publication and mention of the grant of the patent: **20.10.2010**
(21) Application number: 08001803.9
(22) Date of filing: 31.01.2008
(51) Int. Cl.: G01N 33/543, G01N 33/553

(54) **A method for production of a surface plasmon resonance device.**
Verfahren zur Herstellung einer Vorrichtung zur Oberflächenplasmonenresonanz.
Procédé de production d'un dispositif de resonance plasmonique de surface.

(30) Priority: 31.01.2007 JP 2007020349; 17.04.2007 JP 2007108259; 28.09.2007 JP 2007254294
(43) Date of publication of application: 06.08.2008
(73) Proprietor: FUJIFILM Corporation, Minato-ku Tokyo (JP)
(72) Inventor: Saitoh, Yukou, Ashigarakami-gu Kanagawa 258-8577 (JP); Kuruma, Koji, Ashigarakami-gu Kanagawa 258-8577 (JP); Nishimi, Taisei, Ashigarakami-gu Kanagawa 258-8577 (JP); Ohta, Hiroyuki, Ashigarakami-gu Kanagawa 258-8577 (JP); Ezoe, Toshihide, Woodbridge, CT 06525 (US)
(74) Representative: HOFFMANN EITLE

(56) References cited:
- WO-A-99/40954
- JP-A- 2006 058 072
- US-A- 5 436 161
- US-A1- 2006 223 113
- LOEFAAS S ET AL: "A NOVEL HYDROGEL MATRIX ON GOLD SURFACES IN SURFACE PLASMON RESONANCE SENSORS FOR FAST AND EFFICIENT COVALENT IMMOBILIZATION OF LIGANDS" JOURNAL OF THE CHEMICAL SOCIETY, CHEMICAL COMMUNICATIONS, CHEMICAL SOCIETY. LETCHWORTH, GB, no. 21, 1990, pages 1526-1528, XP008050238 ISSN: 0022-4936
- XIA NAN ET AL: "A streptavidin linker layer that functions after drying." LANGMUIR : THE ACS JOURNAL OF SURFACES AND COLLOIDS 27 APR 2004, vol. 20, no. 9, 27 April 2004 (2004-04-27), pages 3710-3716, XP002478595 ISSN: 0743-7463
- VERMETTE PATRICK ET AL: "Immobilization and surface characterization of NeutrAvidin biotin-binding protein on different hydrogel interlayers." JOURNAL OF COLLOID AND INTERFACE SCIENCE 1 MAR 2003, vol. 259, no. 1, 1 March 2003 (2003-03-01), pages 13-26, XP002478596 ISSN: 0021-9797

## Description

### Technical Field

The present invention relates to a method for production of a surface plasmon resonance measurement device containing a physiologically active substance-immobilized substrate.

### Background Art

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a changes in frequency of a crystal due to adsorption or desorption of a substance on gold electrodes of a quartz crystal (device), In addition, the ultra-fine particle surface (nm level) of gold is functionalized, and physiologically active substances are immobilized thereon. Thus, a reaction to recognize specificity among physiologically active substances is earned out, thereby detecting a substance associated with a living organism from sedimentation of gold fine particles or sequences.

In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.

A method comprising allowing a physiologically active substance to have an electric charge opposite to the electric charge of a substrate and localizing the physiologically active substance at a high concentration in proximity to the substrate by use of electrostatic attraction to bond them has been used as a method of immobilizing a physiologically active substance onto a substrate through a covalent bond. According to this method, the physiologically active substance can be immobilized at a high concentration and uniformly onto a substrate for a sensor. However, this method requires using a large amount of a solution. The pH of the solution is limited to a range capable of being charged and concentrated. There has been concern that the physiologically active substance is inactivated.

Further, U.S. Patent No. 5,456,161 and Journal of Colloid and Interface Science 259 (2003) 13-26 disclose the immobilization of a physiologically active substance onto a substrate. In both of these methods, the immobilization is performed in a solution. It has been known in the art to dry a physiologically active substance and to apply a solution with a spin coater or dispenser. However, because a physiologically active substance immobilized on a substrate through a covalent bond is more likely to be inactivated due to the covalent bond, these has been concern so far that application in a small amount places the physiologically active substance in a state that easily permits drying, or causes the physiologically active substance to be spontaneously dried. In U.S. Patent No. 5,436,161, the immobilization of a physiologically active substance is performed in a channel. Therefore, drying or application in a small amount cannot be applied thereto. In Journal of Colloid and Interface Science 259 (2003) 13-26, the immobilization method of a physiologically active substance is not a covalent bond Therefore, even if drying or application is performed, there is a high possibility that the bond is dissociated at a washing step.

WO 99/40954 discloses a method of covalently immobilizing a bio-active agent onto a substrate, which may be a metal substrate. An aqueous layer of a bio-active layer is provided onto a substrate having an immobilizing layer. Then, the aqueous layer is dried to covalently bind said agent to said substrate.

### Disclosure of the Invention

An object to be attained by the present invention is to provide a method for production of a surface Plasmon resonance measurement device wherein the inactivation of a physiologically active substance can be prevented and/or a physiologically active substance can be immobilized at a high concentration onto a substrate surface without use of electrostatic attraction.

The present inventors have found that a physiologically active substance can be immobilized at a high concentration onto a substrate surface without use of electrostatic attraction by forming a thin film of a solution containing a physiologically active substance onto a sensor substrate and further drying and concentrating the solution. Furthermore, the present inventors have found that a physiologically active substance can be immobilized uniformly by forming a thin film of a solution containing a physiologically active substance and shortening a drying time. The present invention has been completed on the basis of these findings.

Specifically, the present invention provides a method according to claim 1.

Preferably, the concentration of the solution containing a physiologically active substance is between 0.1 mg/ml and 10 mg/ml.

Preferably, the concentration of the solution containing a physiologically active substance is between 1 mg/ml and 10 mg/ml.

Preferably, the layer for immobilizing a physiologically active substance is made of a hydrophilic polymer, hydrophobic polymer, self-assembled membrane-forming molecule, or combination thereof.

Preferably, the hydrophilic polymer is activated to be covalently bound with the physiologically active substance.

Preferably, the thickness of the hydrophilic polymer layer is between 1 nm and 300 nm.

Preferably, the hydrophilic polymer comprises a dextran derivative, cellulose compound, polyacrylic acid derivative, or polyvinyl alcohol derivative.

Preferably, the solution containing a physiologically active substance, which has been applied onto the surface of the metal substrate, is dried under conditions where the temperature difference between dry-bulb and wet-bulb temperatures is 7°C or more.

Preferably, the solution containing a physiologically active substance, which has been applied onto the surface of the metal substrate, is dried under conditions where the temperature difference between dry-bulb and wet-bulb temperatures is 10°C or more.

Preferably, the solution containing a physiologically active substance, which has been applied onto the surface of the metal substrate, is dried under conditions where the temperature difference between dry-bulb and wet-bulb temperatures is 13.5°C or more.

Preferably, the step of applying, to a metal substrate having a layer for immobilizing a physiologically active substance, a solution containing a physiologically active substance capable of forming a covalent bond with a molecule constituting the layer for immobilizing a physiologically active substance is performed in an environment where the temperature difference between dry-bulb and wet-bulb temperatures is 7°C or more.

Preferably, the step of applying, to a metal substrate having a layer for immobilizing a physiologically active substance, a solution containing a physiologically active substance capable of forming a covalent bond with a molecule constituting the layer for immobilizing a physiologically active substance is performed in an environment where the temperature difference between dry-bulb and wet-bulb temperatures is 10°C or more.

Preferably, the step of applying, to a metal substrate having a layer for immobilizing a physiologically active substance, a solution containing a physiologically active substance capable of forming a covalent bond with a molecule constituting the layer for immobilizing a physiologically active substance is performed in an environment where the temperature difference between dry-bulb and wet-bulb temperatures is 13.5°C or more.

Preferably, after the solution containing a physiologically active substance, which has been applied onto the surface of the metal substrate, is dried, the substrate is left standing in an environment where the temperature difference between dry-bulb and wet-bulb temperatures is 7°C or more.

Preferably, after the solution containing a physiologically active substance, which has been applied onto the surface of the metal substrate, is dried, the substrate is left standing in an environment where the temperature difference between dry-bulb and wet-bulb temperatures is 10°C or more.

Preferably, after the solution containing a physiologically active substance, which has been applied onto the surface of the metal substrate, is dried, the substrate is left standing in an environment where the temperature difference between dry-bulb and wet-bulb temperatures is 13.5°C or more.

Preferably, the solution containing a physiologically active substance is applied thereto at an average liquid film thickness of 300 µm or less.

Preferably, the solution containing a physiologically active substance is applied thereto at an average liquid film thickness of 20 µm or less.

Preferably, the solution containing a physiologically active substance is applied thereto with a spin coater or dispenser.

Preferably, the physiologically active substance is a protein.

Preferably, the protein is a protein A, protein G, avidins, calmodulin, or antibody.

Preferably, the substrate is a substrate wherein the amount of the hydrophilic polymer immobilized on the substrate is between 3 ng/mm² and 20 ng/mm².

Preferably, the substrate is a substrate wherein the amount of the hydrophilic polymer immobilized on the substrate is between 3 ng/mm² and 15 ng/mm².

Preferably, the substrate is a substrate wherein the amount of the physiologically active substance immobilized is between 1 ng/mm² and 40 ng/mm².

Preferably, the substrate is a substrate wherein the amount of the physiologically active substance immobilized is between 1 ng/mm² and 20 ng/mm².

The method of the present invention can produce a sensor substrate with the uniform amount of a physiologically active substance immobilized and can reduce the usage of the physiologically active substance used in production. Moreover, the method of the present invention can arbitrarily set the pH of a solution containing a physiologically active substance, which is used in production, and can therefore prevent the inactivation of the physiologically active substance on a sensor substrate surface.

### Brief Description of the Invention

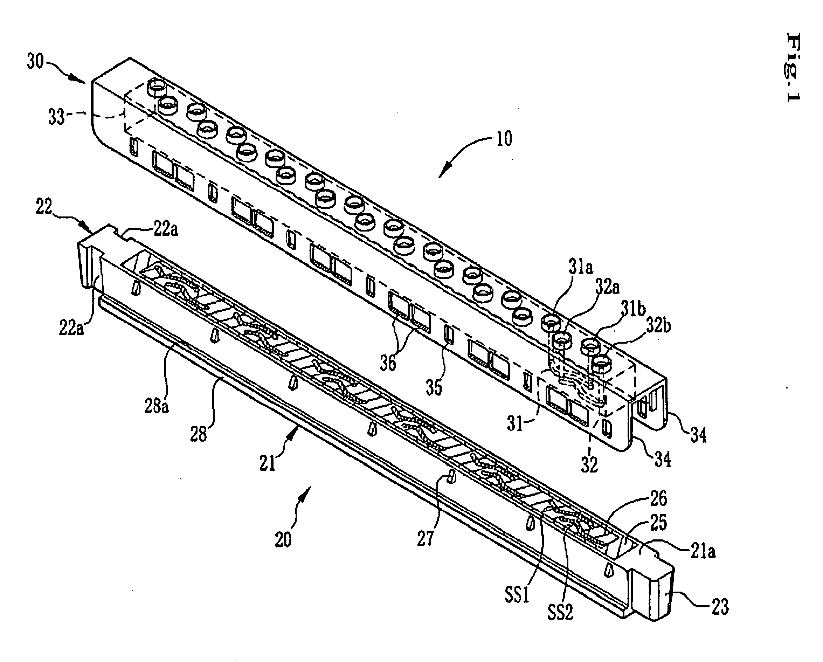

Figure 1 shows an exploded perspective view of a sensor unit

### Preferred Embodiments of the Invention

Hereinafter, the embodiments of the present invention will be explained.

The method according to the present invention is characterized by comprising the steps of: applying, to a metal substrate having a layer for immobilizing a physiologically active substance, a solution containing a physiologically active substance capable of forming a covalent bond with a molecule constituting the layer for immobilizing a physiologically active substance; and then drying the solution within 10 minutes in accordance with claim 1.

In the method of the present invention, a physiologically active substance immobilized on a substrate through a covalent bond is not inactivated even when dried. Moreover, because the physiologically active substance is immobilized on the substrate surface through a covalent bond, the physiologically active substance is not dissociated therefrom at a washing step. Furthermore, when a physiologically active substance is immobilized in a solution, the immobilization cannot be achieved unless its pH is controlled. However, in the method according to the present invention (drying method), immobilization can be achieved at pH preferable for a physiologically active substance. Moreover, the method of the present invention can immobilize a physiologically active substance by applying it in a small amount. Thus, the method of the present invention can reduce the usage of a solution and can therefore reduce cost.

The features of the present invention are summarized as follows: a method using a microchannel of a conventional technique requires a physiologically active substance in large amounts because a solution containing a physiologically active substance must be allowed to flow therein at a step. By contrast, in the method of the present invention, a small amount of a physiologically active substance suffices as a usage because an application method with a spin coater or dispenser can be used. In the method of the present invention, a physiologically active substance is not inactivated because the pH of a solution containing a physiologically active substance does not have to be controlled. It has heretofore been considered that a physiologically active substance is inactivated when dried. However, a physiologically active substance was not inactivated even when dried. Moreover, in the method of the present invention, quantitative and kinetic evaluation errors can be minimized because a film of the physiologically active substance is uniform.

The physiologically active substaace-immobilized substrate which is produced by the production method of the present invention can be used as a biosensor. The biosensor is a surface plasmon resonance measurement device. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured. The physiologically active substance-immobilized substrate of the present invention is explained below.

### (1) Substrate

In the present invention, a physiologically active substance is coated onto the surface of a metal substrate having a layer for immobilizing a physiologically active substance. In the present invention, A metal surface or metal film can be used as a substrate. A metal constituting the metal surface or metal film is not particularly limited, as long as surface plasmon resonance is generated because the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above carrier, an interstinal layer consisting of chrome may be provided between the carrier and a metal layer.

The film thickness of a metal film is not limited. The thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method.

The metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate. The substrate is used for a surface plasmon resonance biosensor, and examples of a substrate include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The aforementioned substrate is immobilized on the dielectric block of a measurement unit and is unified therewith to construct a measurement chip. This measurement chip may be exchangeably formed. An example will be given below.

Figure 1 is an exploded perspective view of a sensor unit 10 used in a measurement that utilizes SPR. The sensor unit 10 is composed of a total internal reflection prism (optical block) 20 that is a transparent dielectric body and a flow channel member 30 equipped on the total internal reflection prism 20. The flow channel member 30 has two types of flow channels, namely, a first flow channel 31 located on the back side of the figure and a second flow channel 32 located on the front side of the figure. When the sensor unit 10 is used in measurement, the two types of flow channels 31 and 32 are used in combination to measure a single sample. However, the details will be described later. In the flow channel member 30, six flow channels 31 and six flow channels 32 are established in the longitudinal direction, so that six samples can be measured in a single sensor unit 10. It is to be noted that the number of either the flow channel 31 or 32 is not limited to six, but that it may be 5 or less, or 7 or more.

The total internal reflection prism 20 is composed of a prism main body 21 formed in a long trapezoidal shape, a gripper 22 established at one end of the prism main body 21, and a projecting portion 23 established at the other end of the prism main body 21. This total internal reflection prism 20 is molded by extrusion molding, for example. The prism main body 21, the gripper 22, and the projecting portion 23 are integrally molded.

The prism main body 21 has a substantially trapezoidal longitudinal section wherein the lower base is longer than the upper base. Light irradiated from the lateral side of the bottom is gathered to an upper surface 21a. A metal film (thin film layer) 25 for exciting SPR is established on the upper surface 21a of the prism main body 21. The shape of the metal film 25 is rectangular such that it faces the flow channels 31 and 32 of the flow channel member 30. The metal film 25 is molded by an evaporation method, for example. The Metal film 25 is made of gold, silver, or the like, and the thickness thereof is 50 nm, for example. The thickness of the metal film 25 is selected as appropriate, depending on the material of the metal film 25, the wavelength of light irradiated during the measurement, etc.

On the metal film 25, a layer for immobilizing a physiologically active substance 26 is established. The layer for immobilizing a physiologically active substance 26 has a binding group for immobilizing a physiologically active substance. A physiologically active substance is immobilized on the metal film 25 via the layer for immobilizing a physiologically active substance 26.

### (2) A layer for immobilizing a physiologically active substance

The metal substrate is provided with a layer for immobilizing a physiologically active substance. The layer for immobilizing a physiologically active substance can be composed of a self-assembled membrane-forming molecule, hydrophilic polymer, hydrophobic polymer, or combination thereof. It is preferred to use a hydrophilic polymer. According to a particularly preferable aspect, the layer can be composed of a combination of a self-assembled membrane-forming molecule and a hydrophilic polymer. (2-1) Self-assembled membrane-forming molecule

The self-assembled membrane described in the present invention refers to an ultrathin film such as a monomolecular film or LB film, which has an organization with certain order formed by a mechanism possessed by a membrane material itself in a state where detailed control is not applied thereto from outside. This self-assembly forms an orderly structure or pattern over a long distance in non-equilibrium conditions.

A method for coating a metal film with the use of a self-assembled membrane (SAMs) has been actively developed by Professor Whitesides et al. (Harvard University). Details of the method are reported in, for example, Chemical Review, 105, 1103-1169 (2005). When gold is used as a metal, an orientational self-assembled monomolecular film is formed with the use of an alkanethiol derivative represented by the following formula A-1 (in the formula A-1, n represents an integer from 3 to 20, and X represents a functional group) as an organic layer-forming compound based on the van der Waals force between an Au-S bond and an alkyl chain. A self-assembled membrane is formed by a very simple method, wherein a gold substrate is immersed in a solution of an alkanethiol derivative. A self-assembled membrane is formed with the use of a compound (represented by the following formula A-1 where X is NH₂) so that it becomes possible to coat a gold surface with an organic layer comprising an amino group:

**HS(CH₂)ₙX** **A-1**

An alkanethiol having an amino group at the end may be a compound comprising a thiol group and an amino group linked via an alkyl chain (formula A-2) (in the formula A-2, n represents an integer of 3 to 20), or may be a compound obtained by reaction between alkanethiol having a carboxyl group at the end (formula A-3 or A-4 (in the formula A-3, n represents an integer of 3 to 20, and in the formula A-4, n each independently represents an integer of 1 to 20) and a large excess of hydrazide or diamine. The reaction between alkanethiol having a carboxyl group at the end and a large excess of hydrazide or diamine may be performed in a solution state. Alternatively, the alkanethiol having a carboxyl group at the end may be bound to the substrate surface and then reacted with a large excess of hydrazide or diamine.

**HS(CH₂)ₙNH₂** **A-2**

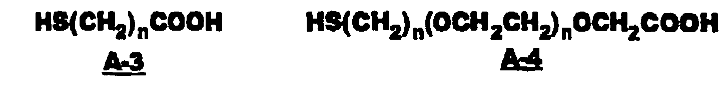

The repeating number of alkyl group of the formulas A-2 to A-4 is preferably 3 to 20, more preferably 3 to 16, and most preferably 4 to 8. If the alkyl chain is short, formation of self-assembled membrane becomes difficult, and if the alkyl chain is long, water solubility decreases and the handling becomes difficult.

Any compound may be used as the diamine used in the present invention. An aqueous diamine is preferable for use in the biosensor surface. Specific examples of the aqueous diamine may include aliphatic diamine such as ethylenediamine, tetraethylenediamine, octamethylenediamine, decamethylenediamine, piperazine, triethylenediamine, diethylenetriamine, triethylenetetraamine, dihexamethylenetriamine, and 1.4-diaminocyclohexane, and aromatic diamine such as paraphenylenediamine, metaphenylenediamine, paraxylylenediamine, metaxylylenediamine, 4,4'-diaminobiphenyl, 4,4'-diaminodiphenylmethane, 4,4'-dianoinodiphenylketone, and 4,4'-diaminodiphenylsulfonic acid. From the viewpoint of increasing the hydrophilicity of the biosensor surface, a compound comprising two amino groups linked via an ethylene glycol unit (formula A-5) may also be used. The diamine used in the present invention is preferably ethylenediamine or the compound represented by the formula A-5 (in the formula A-5, n and m each independently represent an integer of 1 to 20), more preferably ethylenediamine or 1,2-bis(aminoethoxy)ethane (represented by the formula A-5 wherein n=2 and m=1).

**H₂N(CH₂)ₙ(OCH₂CH₂)ₘO(CH₂)ₙNH₂** **A-5**

The alkanethiol having an amino group may form a self assembled membrane by itself or may form a self-assembled membrane by mixing it with another alkanethiol. It is preferred for use in the biosensor surface that a compound capable of suppressing the nonspecific adsorption of a physiologically active substance should be used as the another alkanethiol. The aforementioned Professor Whitesides et al. have investigated in detail self-assembled membrane capable of suppressing the nonspecific adsorption of a physiologically active substance and have reported that a self-assembled membrane formed from alkanethiol having a hydrophilic group is effective for suppressing nonspecific adsorption (Langmuir, 17, 2841-2850, 5605-5620, and 6336-6343 (2001)). In the present invention, any of compounds described in the aforementioned papers may be used preferably as the alkanethiol that forms a mixed monolayer with an alkanethiol having an amino group. In terms of excellent ability to suppress nonspecific adsorption and ease of acquisition, it is preferred that alkanethiol having a hydroxyl group (formula A-6) or alkanethiol having an ethylene glycol unit (formula A-7) (in the formula A-6, n represents an integer of 3 to 20, and in the formula A-7, n and m each independently represent an integer of 1 to 20) should be used as the alkanethiol that forms a mixed monolayer with an alkanethiol having an amino group. Preferably, in the formula A-6, n represents an integer of 5 or more, and further preferably 10 or more, and further preferably 10 to 30, and most preferably 6-16.

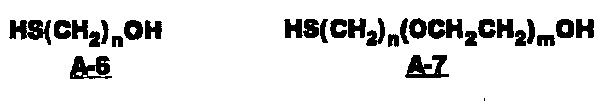

When alkane thiol having an amino group is mixed with another alkane thiol to form a self-assembled membrane, the repeating number of alkyl group of the formulas A-2 to A-4 is preferably 4 to 20, more preferably 4 to 16, and most preferably 4 to 10. Further, the repeating number of alkyl group of the formulas A-6 and A-7 is preferably 3 to 16, more preferably 3 to 12, and most preferably 3 to 8.

In the present invention, it is possible to mix alkanethiol having an amino group and alkanethiol having a hydrophilic group at an arbitrary ratio. However, when the content of alkanethiol having an amino group is low, the amount of actively esterified carboxyl group-containing polymer to be bound decreases. When the content of alkanethiol having a hydrophilic group is low, the capacity for suppression of nonspecific adsorption is reduced. Thus, the mixing ratio of alkanethiol having an amino group to alkanethiol having a hydrophilic group is preferably 1:1 to 1:1,000,000, more preferably 1:1 to 1:1,000, and further preferably 1:1 to 1:10. In view of reduction of steric hindrance upon a reaction with an actively esterified carboxyl group-containing polymer, the molecular length of alkanethiol having an amino group is preferably longer than that of alkanethiol having a hydrophilic group.

As alkanethiol used for the present invention, compounds synthesized based on Abstract, Curr. Org. Chem., 8, 1763-1797 (2004) (Professor Grzybowski, Northwestern University) and references cited therein or a commercially available compound may be used. It is possible to purchase such compounds from Dojindo Laboratories, Aldrich, SensoPath Technologies, Frontier Scientific Inc., and the like. In the present invention, disulfide compounds that are oxidation products of alkanethiol can be used in the same manner as alkanethiol.

### (2-2) Hydrophilic polymer

The hydrophilic polymer that can be used in the present invention can include gelatin, agarose, chitosan, dextran, carrageenan, alginic acid, starch, cellulose, or derivatives thereof, for example, carboxymethyl derivatives, or water-swellable organic polymers, for example, polyvinyl alcohol, polyacrylic acid, polyacrylamide, polyethylene glycol, or derivatives thereof.

The hydrophilic polymer that is used in the present invention includes a synthetic polymer containing a carboxyl group and polysaccharide containing a carboxyl group. Examples of a synthetic polymer containing a carboxyl group include polyacrylic acid, polymethacrylic acid, and copolymers of such acids, including methacrylic acid copolymer, acrylic acid copolymer, itaconic acid copolymer, crotonic acid copolymer, maleic acid copolymer, partially esterified maleic acid copolymer, and a polymer containing a hydroxyl group to which acid anhydride is added described in JP Patent Publication (Kokai) No. 59-53836 A (1984) (page 3, lines 20 to page 6, line 49 of the specification) and JP Patent Publication (Kokai) No. 59-71048 A (1984) (page 3, lines 41 to page 7, line 54 of the specification). A polysaccharide containing a carboxyl group may be extracts from natural plants, microbial fermentation products, enzymatically synthesized products, or chemically synthesized products. Specific examples thereof include hyaluronic acid, chondroitin sulfate, heparin, dermatan sulfate, carboxymethyl cellulose, carboxyethyl cellulose, cellouronic acid, carboxymethyl chitin, carboxymethyl dextran, and carboxymethyl starch. As such polysaccharide containing a carboxyl group, it is possible to use a commercially available compound. Specific examples thereof include carboxymethyldextrans such as CMD, CMD-L, and CMD-D40 (Meito Sangyo Co., Ltd.), sodium carboxymethyl cellulose (Wako Pure Chemical Industries, Ltd.), and sodium alginate (Wako Pure Chemical Industries, Ltd.).

The molecular weight of the hydrophilic polymer used in the present invention is not particularly limited and is generally preferably between 200 and 5,000,000. The further preferable molecular weight of the hydrophilic polymer is between 100,000 and 2,000,000.

The hydrophilic polymer as described above may be immobilized on a substrate via a self-assembled membrane or hydrophobic polymer as described below in the present specification, or can also be formed directly on a substrate from a solution containing a monomer. Furthermore, the hydrophilic polymer can also be cross-linked. The cross-linking of the hydrophilic polymer is obvious to those skilled in the art.

The hydrophilic polymer immobilized to the sensor surface preferably has a thickness of 1 nm to 300 nm in an aqueous solution. Thinner film thickness results in a smaller amount of a physiologically active substance being immobilized. In addition, since a hydrate layer on the sensor surface is thinned, the physiologically active substance itself is denatured to make it difficult to detect the interaction with an analyte. Thicker film thickness results in an obstacle of diffusion of an analyte in the film. In particular, when interaction is detected from the opposite side of the hydrophilic polymer compound-immobilizing face of a sensor substrate, the distance between the detection surface and the interaction-forming portion is large to cause a decrease in detection sensitivity. The thickness of a hydrophilic polymer compound in an aqueous solution can be evaluated by, for example, AFM or ellipsometry.

In the present invention, the amount of the hydrophilic polymer immobilized on the sensor surface is preferably between 3 ng/mm² and 30 ng/mm², and more preferably between 3 ng/mm² and 20 ng/mm², and most preferably between 3 ng/mm² and 15 ng/mm².

As the amount of the hydrophilic polymer immobilized, a value obtained by various film thickness measurement methods can be used. Examples of film thickness measurement methods may include scanning probe microscope (SPM) such as atomic force microscope (AFM) and scanning tunneling microscope (STM); electron microscope such as transmission electron microscope (TEM), scanning electron microscope (SEM) and scanning transmission electron microscope (STEM); quartz crystal microbalance (QCM) method; surface plasmon resonance (SPR) method; attenuated total reflection (ATR) method; infrared spectroscopic method such as external reflection; and ellipsometry method. For example, in SPM, the immobilization amount can be calculated by obtaining difference between the surface irregularity of an area having a hydrophilic polymer immobilized thereon and the surface irregularity of an area having no hydrophilic polymer immobilized thereon. In electron microscope, immobilization amount can be calculated from observation of section of substrate having a hydrophilic polymer immobilized thereon. In infrared spectroscopic method, immobilization amount can be quantified from a calibration curve of absorption strength which is attributed to a hydrophilic polymer. In QCM, SPR and ellipsometry method, an amount of hydrophilic polymer immobilized on a substrate can be determined based on each measurement principle. In the present invention, the film thickness of the immobilized hydrophilic polymer was calculated as mentioned below. Using a spectroscopic ellipsometry (manufactured by FiveLab), an optical constant of the substrate before a hydrophilic polymer is immobilized is determined, and then an optical constant of the substrate in a dry state after a hydrophilic polymer is immobilized is determined so as to determine an optical constant of the hydrophilic polymer layer. When the surface of substrate is composed of several layers, ellipsometry measurement is carried out for formation of each layer, so that an optical constant of each layer can be calculated. Calculation can also be carried out by considering about 2 layers as being single layer. The calculated optical constant of each layer is used as a constant. The difference between the film thickness of the substrate before the immobilization of a hydrophilic polymer and the film thickness of the dry substrate of the hydrophilic polymer after the immobilization of the a hydrophilic polymer, measured by use of monochromatic ellipsometry (He-Ne laser, manufactured by FiveLab) was used as the film thickness of the hydrophilic polymer layer. In this context, the dry state means that water in a liquid state is substantially absent in the hydrophilic polymer layer. Specifically, the dry state is a constant state obtained by standing for 5 minutes or more under temperature and humidity conditions that stabilize the physiologically active substance of the present invention. More specifically, the dry state denotes a state rendered constant by standing for 5 minutes or more at an air temperature of 5°C to 30°C and a humidity of 0% RH to 90%.

### (2-3) Activation of hydrophilic polymer

When a polymer containing a carboxyl group is used as the hydrophilic polymer, the carboxyl group can be activated to thereby immobilize the polymer onto a self-assembled membrane-coated substrate. An approach known in the art, for example, a method comprising performing activation with 1-(3-Dimethylaminopropyl)-3 ethylcarbodiimide (EDC) as water-soluble carbodiimide and N-Hydroxysuccinimide (NHS), or a method comprising performing activation with EDC alone, can be used preferably as a method of activating the polymer containing a carboxyl group. The polymer containing the carboxyl group activated by this approach can be reacted with the substrate having an amino group to thereby produce the biosensor of the present invention.

Moreover, another method for activation of a polymer containing a carboxyl groups is a method that uses a nitrogen-containing compound. Specifically, a nitrogen-containing compound represented by the following formula (Ia) or (Ib) [wherein R₁ and R₂ mutually independently denote a carbonyl group, a carbon atom, or a nitrogen atom, which may have a substituent, R₁ and R₂ may form 5- to 6-membered rings via binding, "A" denotes a carbon atom or a phosphorus atom, which has a substituent, "M" denotes an (n-1)-valent element, and "X" denotes a halogen atom] can also be used.

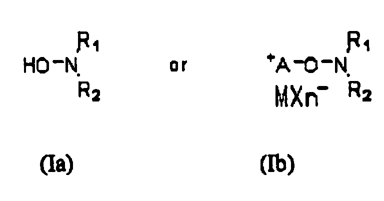

R₁ and R₂ mutually independently denote a carbonyl group, a carbon atom, or a nitrogen atom, which may have a substituent, and preferably R₁ and R₂ form 5- to 6-membered rings via binding. Particularly preferably, hydroxysuccinic acid, hydroxyphthalic acid, 1-hydroxybenzotriazole, 3,4-dihydroxy-3-hydroxy-4-oxo-1,2,3-benzotriazine, and a derivative thereof are provided.

Further preferably, a nitrogen-containing compound represented by the following compound can also be used.

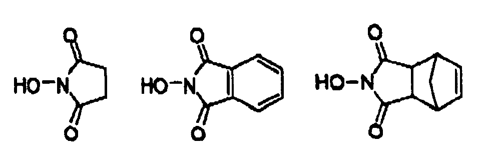

More preferably, a compound represented by the following formula (II) [wherein "Y" and "Z" mutually independently denote CH or a nitrogen atom] can also be used as a nitrogen-containing compound.

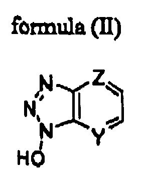

Specifically, the following compounds can be used, for example.

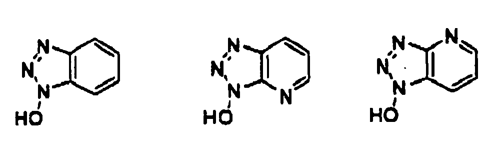

Further preferably, the following compound can also be used as a nitrogen-containing compound.

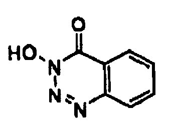

Further preferably, a compound represented by the following formula (III) [wherein "A" denotes a carbon atom or a phosphorus atom, which has a substituent, "Y" and "Z" mutually independently denote CH or a nitrogen atom, "M" denotes a (n-1)-valent element, and X denotes a halogen atom] can also be used as a nitrogen-containing compound.

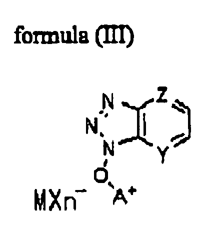

A substituent of a carbon atom or a phosphorus atom denoted by "A" is preferably an amino group having a substituent. Furthermore, a dialkylamino group such as a dimethylamino group or a pyrrolidino group is preferable. Examples of an (n-1)-valent element denoted by "M" include a phosphorus atom, a boron atom, and an arsenic atom. A preferable example of such (n-1)-valent element is a phosphorus atom. Examples of a halogen atom denoted by "X" include a fluorine atom, a chlorine atom, a bromine atom, and an iodine atom. A preferable example of such halogen atom is a fluorine atom.

Furthermore, specific examples of such nitrogen-containing compound represented by formula (III) include the following compounds, for example.

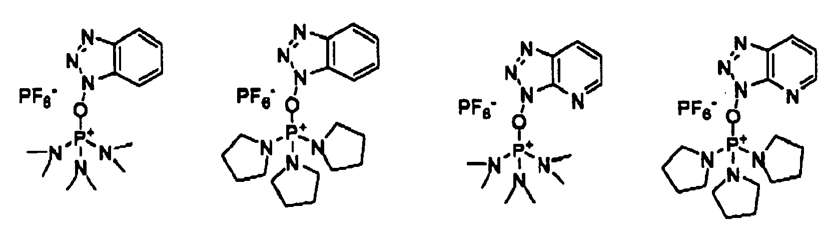

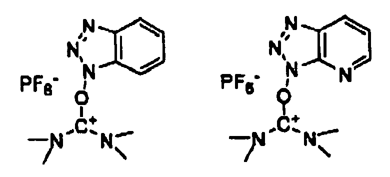

Further preferably, a compound represented by the following formula (IV) [wherein "A" denotes a carbon atom or a phosphorus atom, which has a substituent, "M" denotes an (n-1)-valent element, and "X" denotes a halogen atom] can also be used as a nitrogen-containing compound.

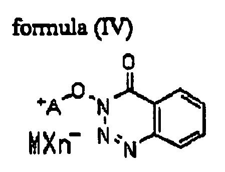

Specifically, the following compound can be used, for example.

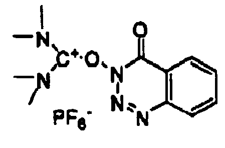

Moreover, as a method for activating a polymer containing carboxyl group, the use of a phenol derivative having an electron-withdrawing group is also preferable. Furthermore, the σ value of the electron-withdrawing group is preferably 0.3 or higher. Specifically, the following compounds or the like can also be used.

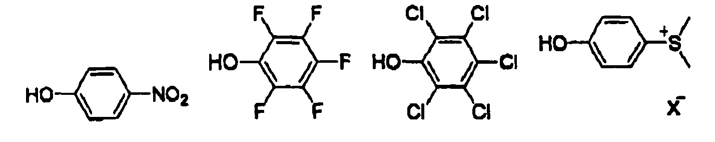

Furthermore, a carbodiimide derivative can further be used separately for such method for activating a polymer containing carboxyl group in combination with the above compounds. Preferably, a water-soluble carbodiimide derivative can be used in combination with such compounds. Further preferably, the following compound (1-Ethyl-3-(3-dimethylaminopropyl)carbodiimide, hydrochloride) can be used in combination with such compounds.

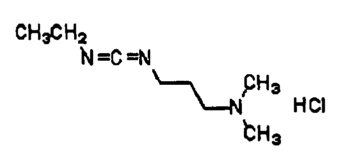

The above carbodiimide derivative and nitrogen-containing compound or phenol derivative can be used not only in such manner, but also independently, if desired. Preferably, a carbodiimide derivative and a nitrogen-containing compound are used in combination.

Furthermore, the following compound can also be used in such method for activating carboxyl groups. The compound can be used independently and can also be used in combination with a carbodiimide derivative, a nitrogen-containing compound, and/or a phenol derivative.

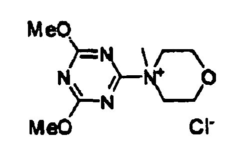

As a method of activating carboxylic acid in the polymer containing a carboxyl group, the method described in Japanese Patent Application No. 2004-238396 (JP Patent Publication (Kokai) No.2006-58071A), paragraphs [0011] to [0022] (that is, a method of activating a carboxyl group existing on the surface of a substrate using any compound selected from a uronium salt, a phosphonium salt, and a triazine derivative, which have a specific structure, so as to form a carboxylic amide group) and the method described in Japanese Patent Application No. 2004-275012 (JP Patent Publication (Kokai) No.2006-90781A), paragraphs [0011] to [0019] (that is, a method, which comprises activating a carboxyl group existing on the surface of a substrate using a carbodiimide derivative or a salt thereof, converting the resultant to an ester using any compound selected from a nitrogen-containing hetero aromatic compound having a hydroxyl group, a phenol derivative having an electron attracting group, and an aromatic compound having a thiol group, and allowing the ester to react with amine, so as to form a carboxylic amide group) can preferably be used.

It is to be noted that the aforementioned uronium salt, phosphonium salt, and triazine derivative, which have a specific structure, described in Japanese Patent Application No. 2004-238396(JP Patent Publication (Kokai) No.2006-58071A), mean the uronium salt represented by the following formula 1, the phosphonium salt represented by the following formula 2, and the triazine derivative represented by the following formula 3, respectively.

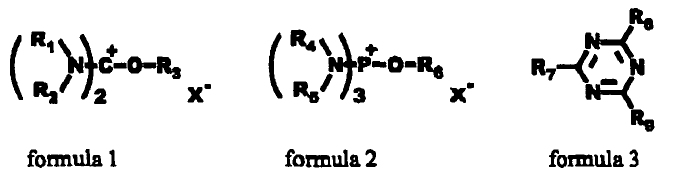

(in formula 1, each of R₁ and R₂ independently represents an alkyl group containing 1 to 6 carbon atoms, or R₁ and R₂ together form an alkylene group containing 2 to 6 carbon atoms, which forms a ring together with an N atom, R₃ represents an aromatic ring group containing 6 to 20 carbon atoms, or a hetero ring group containing at least one heteroatom, and X⁻ represents an anion; in formula 2, each of R₄ and R₅ independently represents an alkyl group containing 1 to 6 carbon atoms, or R₄ and R₅ together form an alkylene group containing 2 to 6 carbon atoms, which forms a ring together with an N atom, R₆ represents an aromatic ring group containing 6 to 20 carbon atoms, or a hetero ring group containing at least one heteroatom, and X⁻ represents an anion; and in formula 3, R₇ represents an onium group, and each of R₈ and R₉ independently represents an electron donating group.)

### <Coating of a hydrophilic polymer on a substrate>

In the present invention, a polymer containing an actively esterified carboxyl group, which is in the form of a solution, may be allowed to react with a substrate. Otherwise, it may also be allowed to react therewith in a state where a thin film has been formed on a substrate by methods such as spin coating. The reaction is preferably carried out in a state where a thin film has been formed is preferable.

As stated above, the polymer containing an actively esterified carboxyl group in the present invention may be preferably allowed to react with a substrate in the state of a thin film. As a method for forming a thin film on a substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method. These methods for forming a thin film are described in "Progress in Coating Technology (Coating Gijutsu no Shinpo)" written by Yuji Harazaki, Sogo Gijutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinka-suru Organic Thin Film: Seimaku hen)" Sumibe Techno Research Co., Ltd. (2004); "Polymer Surface Processing Technology (Polymer Hyomen Kako Gaku)" written by Akira Iwamori, Gihodo Shuppan Co., Ltd. (2005); and the like. As the method for forming a thin film on a substrate of the present invention, a spray coating method, a dispensing method or a spin coating method is preferable. Further, a spin coating method is more preferable. This is because it allows a coating film having a controlled film thickness to be readily produced.

The spray coating method is a method wherein a substrate is moved with an ultra-atomized polymer solution sprayed onto the substrate to thereby uniformly coat the polymer solution onto the substrate. When the trigger of a spray gun is pulled, an air valve and a needle valve are simultaneously opened. The polymer solution is ejected in the form of a fine mist from a nozzle, and this polymer solution in the form of a fine mist is further ultra-atomized by air ejected from an air cap located at the end of the nozzle. A thickness-controlled polymer film is easily produced by forming the coating film of the ultra-atomized polymer solution on the substrate surface, followed by the evaporation of the solvent. The thickness of the polymer thin film can be controlled on the basis of the concentration of the polymer solution, the moving speed of the substrate, and so on.

The dispensing method is a method wherein a polymer solution is uniformly applied onto a substrate by use of a dispenser that discharges an application solution in a constant amount. While the polymer solution is discharged from a discharge apparatus such as a syringe pump, a discharge port can be moved at a constant speed on the substrate to thereby uniformly apply the polymer solution in arbitrary sites on the substrate. This method is preferable because the amount of the solution applied can be render uniform by maintaining the regular spacing between the substrate and the discharge port, and furthermore, reduction in the usage of the application solution and improvement in drying speed can be achieved by keeping the spacing as small as possible to thereby decrease the thickness of the application solution.

The spin coating method is a method wherein a polymer solution is added dropwise onto a substrate placed horizontally, which is then spun at a high speed to thereby uniformly coat the polymer solution onto the whole surface of the substrate through a centrifugal force. A thickness-controlled polymer film is easily produced with the scattering of the polymer solution through a centrifugal force and the evaporation of the solvent. The thickness of the polymer thin film can be controlled on the basis of the revolution speed, the concentration of the polymer solution, the vapor pressure of the solvent, and so on. In the present invention, the revolution speed during spin coating is not particularly limited. If the revolution speed is too small, the solution remains on the substrate. If the revolution speed is too large, an available apparatus is restricted.

### (2-5) Hydrophobic polymer

The hydrophobic polymer which can be used in the present invention is a polymer compound lacking water-absorbing properties and having solubility (25°C) in water of 10% or less, more preferably 1% or less, and most preferably, 0.1% or less.

Hydrophobic monomers that form such hydrophobic polymer compound can be arbitrarily selected from among vinyl esters, acrylic acid esters, methacrylic acid esters, olefins, styrenes, crotonic acid esters, itaconic acid diesters, maleic acid diesters, fumaric acid diesters, alkyl compounds, vinyl ethers or vinyl ketones. Such hydrophobic polymer may be a homopolymer comprising one type of monomer, or a copolymer comprising two or more types of monomer.

Examples of such hydrophobic polymer compound that is preferably used in the present invention include polystyrene, polyethylene, polypropylene, polyethylene terephthalate, polyvinylchloride, polymethyl methacrylate, polyester, and nylon.

A substrate can be coated with such hydrophobic polymer by a conventional method such as spin coating, air-knife coating, bar coating, blade coating, slide coating, or curtain coating. Coating can also be performed by a spray method, an evaporation method, a cast method or a dipping method.

The coating thickness of the hydrophobic polymer is not particularly limited and is preferably 0.1 nm to 500 nm, and particularly preferably 1 nm to 300 nm. The molecular weight of the hydrophobic polymer is not particularly limited and is preferably between 10,000 and 50,000,000.

### (3) Immobilization of a physiologically active substance on a layer for immobilizing a physiologically active substance

It is preferred that the layer for immobilizing a physiologically active substance should have a functional group capable of immobilizing a physiologically active substance. For example, when the layer for immobilizing a physiologically active substance is composed of a hydrophilic polymer having a carboxyl group, this carboxyl group can be activated and then reacted with a physiologically active substance having an amino group to thereby immobilize the physiologically active substance onto the hydrophilic polymer. The activation of the carboxyl group for immobilizing the physiologically active substance onto the layer for immobilizing a physiologically active substance can be performed in the same way as in the method described above in (2-3) Activation of hydrophilic polymer in the present specification.

In the present invention, onto the surface of a metal substrate having a layer for immobilizing a physiologically active substance, a solution containing a physiologically active substance capable of forming a covalent bond with a molecule constituting the layer for immobilizing a physiologically active substance is applied. Then, the solution is dried to thereby form a uniform film on the substrate surface. In this procedure, the physiologically active substance is immobilized on the surface of the metal substrate through a covalent bond with the molecule constituting the layer for immobilizing a physiologically active substance.

In the present invention, an aspect is possible, wherein in addition to a functional group such as a carboxyl group or amino group as the functional group capable of immobilizing a physiologically active substance, for example, a physiologically active substance such as a biotin-binding protein (avidin, streptavidin, NeutrAvidin, etc.), protein A, protein G, antigen, or antibody (e.g., tag antibodies known in the art such as anti-GST antibodies) is immobilized in advance, and on this physiologically active substance, a physiologically active substance as described below is further immobilized. Alternatively, when an immobilizing layer comprising an alkane introduced in a polymer is used, a physiologically active substance having a membrane structure, such as lipid, can be immobilized therein. Moreover, depending on applications, the present invention is adaptable to diverse proteins by adjusting a polymer chain length, polymer thickness, polymer density, or the amount of a reactive group introduced into the polymer. Moreover, when NTA (nitrilotriacetic acid) or the like is introduced as an immobilizing group into a polymer, a His-tag ligand or the like can be immobilized via a metal chelate.

### (4) Physiologically active substance which can be used in the present invention

A physiologically active substance immobilized on the metal substrate in the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus,* antibiotic, narcotic drug, cocaine, heroin, crack or the like, there can be used, for example, an anti-atrazine antibody, anti-kanainycin antibody, anti-metamphetamine antibody, or antibodies against 0 antigens 26, 86, 55, 111 and 157 among enteropathogenic *Escherichia coli.*

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor. Examples of an immunoglobulin-binding protein used herein may include protein A, protein G, and a rheumatoid factor (RF).

As a sugar-binding protein, for example, lectin is used.

Examples of fatty acid or fatty acid ester may include stearic acid, arachidic acid, behenic acid, ethyl stearate, ethyl arachidate, and ethyl behenate.

Among these physiologically active substances, it is preferred to use protein, and it is more preferred to use protein A, protein G, avidins, calmodulin, or antibody.
With regard to the concentration of a solution (an applied solution) that contains physiologically active substance, it is preferable that the concentration of physiologically active substance immobilized on a substrate surface be high. The aforementioned concentration is preferably between 0.1 mg/ml and 10 mg/ml, and more preferably between 1 mg/ml and 10 mg/ml, although it depends on the type of physiologically active substance.

### (5) Applying and drying of physiologically active substance

In the process of drying such a solution that contains physiologically active substance, the physiologically active substance tend to be precipitated from the peripheral portion of the applied solution, or from a portion in which the liquid remains immediately before the applied solution is dried. Thereby, the quantities of physiologically active substance immobilized on the substrate surface vary. This is not preferable. In order to uniform the quantities of physiologically active substance immobilized on the substrate surface, it is preferable that the viscosity of the applied solution be set at high, so far as it does not inhibit the binding of the physiologically active substance to the substrate surface. By setting the viscosity of the applied solution at high, the movement of the physiologically active substance contained in the applied solution in the horizontal direction towards the substrate surface can be suppressed during the drying process. As a result, variations in the quantities of physiologically active substance immobilized can be suppressed. The viscosity of the applied solution is preferably maintained at 0.9 cP or more during the drying process.

The term "drying process" is used in the present invention to comprise two steps of an application step of applying a solution containing physiologically active substance, and an intentional drying step whereby the speed of drying the aforementioned solution is increased by heating or air-blowing after the application step. An increase in the drying rate of the solution that contains the physiologically active substance (coating solution) is also effective for suppressing variation in the amount of the physiologically active substance immobilized. The drying rate is increased, and the drying process is sufficiently rapidly completed with respect to the movement of the physiologically active substance in the horizontal direction. Thereby, the drying process is completed before the physiologically active substance substantially moves, so that the variation can be suppressed. The type of a method of increasing such a drying speed is not particularly limited. Examples of such a method include a method of increasing the temperature of the applied solution or a temperature in dry environment, a method of adding evaporation energy by irradiation with infrared ray or laser, a method of decreasing a solvent vapor pressure during the drying process by air blowing or the like, and a method of enlarging an evaporation area with respect to the amount of the applied solution by applying the solution to form a thin layer. In particular, when such an applied solution contains water, a drying speed is increased by performing the drying process in an environment wherein there is a great difference between a dry-bulb temperature and a wet-bulb temperature. The drying process is carried out in an environment wherein the difference between such a dry-bulb temperature and a wet-bulb temperature is preferably 7°C or more, more preferably 10°C or more, and further more preferably 13.5°C or more. In addition, taking into consideration a production process, the time required for the drying process is preferably between 0.1 seconds and 10 minutes, more preferably between 1 seconds and 5 minutes, and particularly preferably between 0.1 seconds and 1 minute.

In the present Invention, it is preferred that the application step and the intentional drying step should be performed in an environment where the temperature difference between dry-bulb and wet-bulb temperatures is 7°C or more, more preferably, in an environment where the temperature difference between dry-bulb and wet-bulb temperatures is 10°C or more, even more preferably, in an environment where the temperature difference between dry-bulb and wet-bulb temperatures is 13.5°C or more.

An example of a method of applying a solution that contains physiologically active substance is a method particularly using a dispenser that quantitatively discharges a solution to be applied. In the dispenser, a solution may be stocked in syringe and then discharged. Also, the solution may be discharged by pipette. Use of pipette is particularly preferred, since the amount of a physiologically active substance remaining in the dispenser can be reduced. The discharge port of the dispenser is moved on a substrate at a certain speed at certain intervals, so that the solution can be uniformly applied at any given sites on the substrate. When the solution is applied using a dispenser, the interval between the substrate and the discharge port is extremely narrowed, and the thickness of the applied solution is reduced, so that the thickness of physiologically active substance can be uniformed. Further, the drying speed can also be increased. Thus, the use of such a dispenser is preferable. Another preferred method of applying a solution that contains physiologically active substance is spin coating. This method is particularly preferably applied when the thickness of the applied film is reduced. In this method, since the drying process is carried out after a solution having a uniformed thickness has been formed, it is preferable to prevent evaporation of the solution during rotation of a spin coater. If a method of placing a substrate in a hermetically sealed vessel or the like during rotation is applied to maintain the concentration of a solvent existing around the substrate at high, the drying speed can be controlled before and after formation of a thin film during rotation. Thus, this method is particularly preferable. It is preferred that drying is carried out under a condition where temperature and humidity are kept constant, after this coating process. Also, it is preferred to dry a solution of physiologically active substance by air knife method.

In the present invention, a drying method at the "intentional drying step" also encompasses, in addition to the drying by heating or ventilation described above, a drying method using a spin coating method or air knife method.

Furthermore, in the present invention, it is preferred that after the solution containing a physiologically active substance, which has been applied onto the surface of the metal substrate, is dried (after the application step and the intentional drying step), the substrate should be left standing in an environment where the temperature difference between dry-bulb and wet-bulb temperatures is 7°C or more, more preferably, in an environment where the temperature difference between dry-bulb and wet-bulb temperatures is 10°C or more, even more preferably, in an environment where the temperature difference between dry-bulb and wet-bulb temperatures is 13.5°C or more. In this context, after the application step, the intentional drying step is omitted, and the standing step may be performed. It is preferred that the substrate should be left standing for 1 second to 24 hours, more preferably, for 1 minute to 6 hours, even more preferably, for 5 minutes to 2 hours, most preferably, for 20 minutes to 1 hour, as a time at the standing step.

In the most preferable aspect of the present invention, the step of applying, to a metal substrate having a layer for immobilizing a physiologically active substance, a solution containing a physiologically active substance capable of forming a covalent bond with a molecule constituting the layer for immobilizing a physiologically active substance is performed in an environment where the temperature difference between dry-bulb and wet-bulb temperatures is 7°C or more (more preferably 10°C or more, even more preferably 13.5°C or more). Then, the solution is dried in an environment where the temperature difference between dry-bulb and wet-bulb temperatures is 7°C or more (more preferably 10°C or more, even more preferably 13.5°C or more). Then, the substrate is left standing in an environment where the temperature difference between dry-bulb and wet-bulb temperatures is 7°C or more (more preferably 10°C or more, even more preferably 13.5°C or more). As a result, the physiologically active substance-immobilized substrate can be produced.

When the interaction of physiologically active substance with test substances is detected, variations in the quantities of physiologically active substance immobilized on the sensor surface cause an error in quantitative and kinetic evaluation of such an interaction. In order to keep such an error to a minimum, the quantities of physiologically active substance immobilized are preferably uniformed. A CV value (coefficient variation) (standard deviation/mean value), which indicates variations in the quantities of physiologically active substance immobilized on the surface of a substrate used in detection of an interaction, is preferably 15% or less, and more preferably 10% or less. Such a CV value can be calculated based on the quantities of physiologically active substance immobilized on at least two sites, preferably 10 or more sites, and more preferably 100 or more sites on the substrate surface. Uniformity can be evaluated by quantifying the quantities of substances existing on a sensor substrate before and after immobilization of physiologically active substance. However, such uniformity can also be evaluated by fluorescently-labeling substances that have been known to bind to physiologically active substance, immobilizing such fluorescently-labeled substances on a sensor substrate, and then measuring fluorescence intensity using a fluorescence microscope or the like. Moreover, it is also possible to quantify physiologically active substance using an SPR imager, an ellipsometer, TOF-SEMS, an ATR-IR apparatus, etc.

Moreover, in the present invention, it is preferred that the specific amount of the physiologically active substance immobilized should be, when a hydrophilic polymer is used as the layer for immobilizing a physiologically active substance, 0.3 times or more to 3 times or less the amount of the hydrophilic polymer immobilized (ng/mm²) per unit area. The preferable amount of the physiologically active substance immobilized is between 1 ng/mm² and 40 ng/mm², more preferably, between 1 ng/mm² and 20 ng/mm². The amount of the physiologically active substance immobilized with respect to the amount of the hydrophilic polymer immobilized as well as the amount of the physiologically active substance immobilized can be allowed to fall within this range to thereby further enhance the effect of suppressing the inactivation of the physiologically active substance.

The amount of the physiologically active substance immobilized can be measured in the same way as in the calculation method of the amount of the hydrophilic polymer immobilized. In the present invention, the thickness of the immobilized physiologically active substance was calculated according to an ellipsometry method The optical constant of the physiologically active substance-immobilized hydrophilic polymer in a dry state is determined by use of spectroscopic ellipsometry. The calculated optical constant is used as a constant. The difference between the film thickness of the hydrophilic polymer layer in a dry state after the immobilization of the physiologically active substance measured by use of monochromatic ellipsometry and the film thickness of the dry substrate of the hydrophilic polymer before the immobilization of the physiologically active substance was used as the film thickness of the immobilized physiologically active substance. In this context, the dry state means that water in a liquid state is substantially absent in the physiologically active substance-immobilized hydrophilic polymer layer. Specifically, the dry state is a constant state obtained by standing for 5 minutes or more under temperature and humidity conditions that stabilize the physiologically active substance of the present invention. More specifically, the dry state denotes a state rendered constant by standing for 5 minutes or more at an air temperature of 5°C to 30°C and a humidity of 0% RH to 90%.

### (6) Method of use of the substrate

The substrate to which a physiologically active substance is immobilized as described above can be used to detect and/or measure a substance which interacts with the physiologically active substance.

In the present invention, it is preferable to detect and/or measure an interaction between a physiologically active substance immobilized on the sensor substrate and a test substance by a nonelectric chemical method. The non-electrochemical method is a surface plasmon resonance (SPR) measurement technique.

The substrate is used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle, Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light Accordingly, it is necessary to set the light beam in advance such that it enters as p-polarized light.

If the wave number of a surface plasmon is determined from an incident angle causing the attenuated total reflection (ATR), that is, an attenuated total reflection angle (θSP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

When the biosensor is used in surface plasmon resonance analysis, it can be applied as a part of various surface plasmon measurement devices described above.

The biosensor can be used as a biosensor, which has a waveguide structure on the surface of a carrier, for example, and which detects refractive index changes using such a waveguide. In this case, the waveguide structure on the carrier surface has a diffraction grating, and in some cases, an additional layer. This waveguide structure is a planar waveguide body comprising a thin dielectric layer. Light gathered to the waveguide body form is introduced into such a thin layer by total internal reflection. The transmission velocity of the thus introduced light wave (hereinafter referred to as "mode") is indicated as a C/N value. Herein, C indicates the velocity of light in a vacuum, and N indicates an effective refractive index of the mode introduced into the waveguide body. Such an effective refractive index N is determined based on the structure of the waveguide body on one face, and is determined based on the refractive index of a medium adjacent to the thin waveguide layer on the other face. Conduction of a light wave is carried out not only in a thin planar layer, but also by another waveguide structure, and in particular, by a stripped waveguide body. In such a case, the waveguide structure is processed into the shape of a stripped film. It is an important factor for a biosensor that changes in effective refractive indexes N are generated as a result of changes in the medium adjacent to the waveguide layer, and changes in the refractive index and thickness of the waveguide layer itself or an additional layer adjacent to the waveguide layer.

The structure of a biosensor of this system is described in page 4, line 48 to page 14, line 15, and Figures 1 to 8 of JP Patent Publication (Kokoku) No. 6-27703 B (1994), and column 6, line 31 to column 7, line 47, and Figures 9A and 9B of US Patent No. 6,829,073.

For example, in one embodiment, there is a structure whereby a waveguide layer comprising a planar thin layer is established on a substrate (e.g. Pyrex (registered trademark) glass). A waveguide layer and a substrate form together a so-called waveguide body. Such a waveguide layer can be a multilayer laminated body such as an oxide layer (SiO2, SnO2, Ta2O5, TiO2, TiO2-SiO2, HfO2, ZrO2, Al2O3, Si3N4, HfON, SiON, scandium oxide, or a mixture thereof) or a plastic layer (e.g. polystyrene, polyethylene, polycarbonate, etc.). For transmission of light into a waveguide layer as a result of total internal reflection, the refractive index of the waveguide layer must be greater than that of the adjacent medium (for example, a substrate, or an additional layer as described later). A diffraction grating is disposed on the surface of the waveguide layer or in the bosom thereof, which faces to a substrate or a measured substance. Such a diffraction grating can be formed in a carrier according to embossing, holography, or other methods. Subsequently, the upper surface of the diffraction grating is coated with a thin waveguide film having a higher refractive index. The diffraction grating has the functions to focus rays of light incident on the waveguide layer, to discharge the mode already introduced into the waveguide layer, or to transmit a portion of the mode in the travel direction and reflect a portion thereof. The grating area of the waveguide layer is covered with an additional layer. Such an additional layer can be a multilayer film, as necessary. This additional layer is able to have the function to carry out selective detection of a substance contained in a measured substance. In a preferred embodiment, a layer having a detection function can be established on the outermost surface of such an additional layer. As such a layer having a detection function, a layer capable of immobilizing physiologically active substances can be used.

In another embodiment, it is also possible to adopt a structure whereby an array of diffraction grating waveguides is incorporated into wells of a microplate (JP Patent Publication (Kohyo) No. 2007-501432 A). That is to say, if such diffraction grating waveguides are aligned in the form of an array at the bottoms of wells of a microplate, the screening of a drug or chemical substance can be carried out at a high throughput.

In order to detect physiologically active substances existing on the upper layer (detection area) of a diffraction grating waveguide, the diffraction grating waveguide detects incident light and reflected light, so as to detect changes in refractive properties. For this purpose, one or more light sources (e.g. laser or diode) and one or more detectors (e.g. a spectrometer, a CCD camera, or other light detectors) can be used. As a method of measuring changes in refractive indexes, there are two different operational modes, namely, spectroscopy and an angle method. In spectroscopy, broadband beam used as incident light is transmitted to a diffraction grating waveguide, and reflected light is gathered, followed by a measurement with a spectrometer, for example. By observing the spectrum position of a resonant wavelength (peak), changes in refractive indexes on the surface of the diffraction grating waveguide or a periphery thereof, namely, a bond can be measured. On the other hand, in an angle method, light of a nominally single wavelength is gathered such that it generates a certain range of irradiation angle, and it is directed into the diffraction grating waveguide. The reflected light is measured with a CCD camera or other types of light detectors. By measuring the position of a resonance angle reflected by the diffraction grating wavelength, changes in refractive indexes on the surface of the diffraction grating waveguide or a periphery thereof, namely, a bond can be measured.

The present invention will be further specifically described in the following examples.

### EXAMPLE

### Example 1

### (1) Preparation of substrate

Onto the upper surface of a plastic prism obtained by injection-molding ZEONEX (ZEON CORP.), a gold thin film was formed by the following method: the prism was attached to the substrate holder of a sputtering apparatus. After a vacuum (base pressure: 1×10⁻³ Pa or less) was drawn, Ar gas was introduced (1 Pa). While the substrate holder was rotated (20 rpm), RF power (0.5 kW) was applied to the substrate holder for approximately 9 minutes to plasma-treat the prism surface. Next, the Ar gas was stopped, and a vacuum was drawn. Ar gas was introduced again (0.5 Pa). While the substrate holder was rotated (10 to 40 rpm), DC power (0.2 kW) was applied to an 8-inch Cr target for approximately 30 seconds to form a Cr thin film of 2 nm. Next, the Ar gas was stopped, and a vacuum was drawn again. Ar gas was introduced again (0.5 Pa). While the substrate holder was rotated (20 rpm), DC power (1 kW) was applied to an 8-inch Au target for approximately 50 seconds to form an Au thin film of approximately 50 nm.

The sensor stick thus obtained in which the Au thin film was formed was dipped in a 1 mM aqueous solution of 6-Amino-1-hexanethiol, hydrochloride (manufactured by Dojindo Laboratories) at 40°C for 1 hour and washed five times with ultrapure water.

### (2) Active esterification of CMD (carboxymethyldextran)

After 10 g of 1% by weight of a CMD (manufactured by Meito Sangyo Co., Ltd.; molecular weight: 1,000,000, substitution degree: 0.59) solution (carboxyl group amount: 5×10⁻⁴ mol) was dissolved, 10 ml of an aqueous solution containing 1-ethyl-2,3-dimethylaminopropyl carbodiimide 2×10⁻⁵ M) and N-hydroxysuccinimide (5×10⁻⁵ M) was added thereto. The mixture was stirred at room temperature for 1 hour.

### (3) Binding reaction of CMD to substrate

Onto the substrate prepared in (1), 1 mL of the active-esterified CMD solution prepared in (2) was added dropwise by use of a micropipette throughout the prism surface provided with the Au thin film to coat the overall surface therewith. The substrate was secured onto the inner cup of a spin coater (Model 408 (Special), manufactured by Nanometric Technology Inc.) having a closed-type inner cup so that the longitudinal direction of the substrate corresponded to the tangential direction of a circular arc at a position 135 mm in radius from the rotation center. A thin film of active-esterified carboxymethyldextran was formed by spin coating at 1000 rpm for 45 seconds on the substrate having an amino group. After reaction at room temperature for 15 minutes, the substrate was dipped in a 1 N NaOH aqueous solution for 30 minutes and washed five times with ultrapure water to thereby prepare a CMD-surface substrate. When measurement using AFM was performed, its film thickness was 200 nm.

### (4) Preparation of N-avidin-modified substrate

Onto the CMD substrate surface prepared in (3), a Teflon (registered trademark) plate having a circular hole of 5 mm in diameter was pressed to thereby form a cuvette. Into this cuvette, 100 µL of a 1:1 mixed solution of 1-ethyl-2,3-dimethylaminopropyl carbodiimide (400 mM) and N-hydroxysuccinimide (100 mM) was added. The substrate was left standing at room temperature for 7 minutes to active-esterify the CMD substrate surface. Subsequently, the contents in the cuvette were substituted by a buffer obtained by dissolving 3.8 g of sodium tetraborate decahydrate in 1 L of pure water and adjusting the solution to pH 8.5 by the addition of hydrochloric acid (1 mol/L) (hereinafter, abbreviated to a boric acid buffer). After the boric acid buffer in the cuvette was removed with a nitrogen gun, 5 µL of a 5 mg/mL boric acid buffer solution of N-avidin (manufactured by PIECE) (hereinafter, abbreviated to a 5 mg/mL solution of N-avidin) was added dropwise onto the active-esterified CMD surface. Immediately thereafter, the substrate was transferred to an air bath (the difference between dry-bulb and wet-bulb temperatures was 10°C) at 40°C and left standing. As a result, the N-avidin solution was dried after 10 minutes.

The isoelectric point of the N-avidin used in Examples is pH 6.3. The N-avidin has a negative charge at pH higher than pH 6.3 and has a positive charge at pH lower than pH 6.3. Moreover, the CMD substrate and the active-esterified CMD substrate have a negative charge at pH 5.0 or higher.

### (5) Blocking treatment of N-avidin-modifled substrate

To the N-avidin-modified substrate surface in the cuvette prepared in (4), 100 µL of ethanolamine hydrochloride aqueous solution (1 M; hereinafter, abbreviated to a blocking solution) which was adjusted to pH 8.5 by addition of 1 N sodium hydroxide aqueous solution was added. The substrate was left standing at room temperature for 7 minutes. The contents in the cuvette were substituted by a boric acid buffer, then substituted by a 0.1 N sodium hydroxide aqueous solution, and subsequently substituted by a phosphoric acid buffer at pH 7.4 to thereby prepare an N-avidin-modified substrate treated by blocking.

### (6) Evaluation of N-avidin-modified substrate

The amount of the N-avidin immobilized on the N-avidin-modified substrate surface was measured at 120 positions at 0.25-mm spacings on the substrate by use of an SPR imager (manufactured by CWG; SPR imager) to evaluate the average amount of the N-avidin immobilized and the CV value. The results are shown in Table 1. Variations in the immobilized amount are indicated by a CV value = standard deviation of the immobilized amount/an average value of the immobilized amount × 100. 1 RU corresponds to the amount of the N-avidin immobilized on the order of 1 pg/mm².

### Example 2

To the CMD-surface substrate surface of Example 1, 1 mL of a 1:1 mixed solution of 1-ethyl-2,3-dimethylaminopropyl carbodiimide (400 mM) and N-hydroxysuccinimide (100 mM) was added dropwise to coat the CMD surface therewith. The substrate was left standing at room temperature for 7 minutes to active-esterify the CMD substrate surface. Subsequently, the substrate surface was washed with a boric acid buffer. The boric acid buffer was removed with a nitrogen gun.

To the active-esterified CMD-surface substrate, 100 µL of a 5 mg/mL solution of N-avidin was added dropwise by use of a syringe pump. Furthermore, the solution was spread with a spatula made of polypropylene to coat the overall substrate surface therewith. Then, the substrate was placed in a closed container for spin coating. This closed container was secured onto the inner cup of a spin coater (Model 408 (Special), manufactured by Nanometric Technology Inc.) having a closed-type inner cup so that the longitudinal direction of the substrate corresponded to the tangential direction of a circular arc at a position 135 mm in radius from the rotation center (the difference between dry-bulb and wet-bulb temperatures in the spin coater was 10°C). In a state where the substrate was placed in the closed container, the substrate was rotated at 500 rpm for 45 seconds. Then, the substrate was taken out of the closed container. The N-avidin aqueous solution on the substrate surface was dried in approximately 1 second after the substrate was taken out of the closed container.

This N-avidin-modified substrate surface was washed with a 1 N sodium hydroxide aqueous solution and dipped in a blocking solution at room temperature for 7 minutes. Subsequently, the substrate was dipped three times in a boric acid buffer, then dipped three times in a 0.1 N sodium hydroxide aqueous solution, and subsequently dipped three times in a phosphoric acid buffer at pH 7.4 to thereby prepare an N-avidin-modified substrate treated by blocking.

The prepared N-avidin-modified substrate was evaluated by the method of Example 1 except that spacings between positions measured with an ellipsometer were set to 2.5 mm. The results are shown in Table 1.

### Example 3

To a CMD substrate active-esterified by the method described in Example 2, a 5 mg/mL solution of N-avidin was applied within an area of 1 mm in width and 11 cm in length in the center of the substrate by use of a dispenser (FAD320s, dedicated desktop-type dispensing robot with an image recognition function, manufactured by Musashi Engineering, Inc.). When the substrate was left standing under conditions where the difference between dry-bulb and wet-bulb temperatures was 10°C, the solution was dried in 3 minutes. The N-avidin-modified surface prepared by this procedure was evaluated in the same way as in Example 2. The evaluation results are shown in Table 1.

### Comparative Example 1

The contents in the cuvette of the active-esterified CMD substrate surface of Example 1 were substituted by a 10 mM acetic acid aqueous solution (hereinafter, abbreviated to an acetic acid buffer) adjusted to pH 5.0 with a 0.1 N sodium hydroxide aqueous solution. After the acetic acid buffer was removed, 100 µL of a 0.2 mg/mL acetic acid buffer solution of N-avidin (hereinafter, abbreviated to a 0.2 mg/mL solution of N-avidin) was added dropwise to the active-esterified CMD surface. After the substrate was left standing at room temperature for 30 minutes, the 0.2 mg/mL solution of N-avidin in the cuvette was substituted by 100 µL of a blocking solution. The substrate was left standing at room temperature for 7 minutes. The contents in the cuvette were substituted by an acetic acid buffer, then substituted by a 0.1 N sodium hydroxide aqueous solution, and subsequently substituted by a phosphoric acid buffer at pH 7.4 to thereby prepare an N-avidin-modified substrate treated by blocking.

The prepared N-avidin-modifled surface was evaluated in the same way as in Example 1. The results are shown in Table 1.

### Comparative Example 2

An N-avidin-modified substrate was prepared in the same way as in Comparative Example 1 except that a boric acid buffer was used instead of all the acetic acid buffers of Comparative Example 1. The substrate was evaluated in the same way as in Example 1. The results are shown in Table 1.

As shown in Table 1, according to the method of the present invention, the physiologically active substance could be bonded covalently to the substrate surface even without adjusting pH to give an electric charge opposite to that of the substrate for immobilization. Furthermore, a thin film of the physiologically active substance solution could be formed by use of a spin coating method or dispensing method to thereby obtain a uniform physiologically active substance-modified surface.

**Table 1**

| | Average thickness of N-avidin solution [µm] | Electric charge of N-avidin/electric charge of substrate | Amount of N-avidin immobilized (average) [RU] | Variations in amount of N-avidin immobilized |
|---|---|---|---|---|
| Example 1 | 250 | -/- | 11400 | 25 |
| Example 2 | 5 | -/- | 12000 | 3 |
| Example 3 | 20 | -/- | 16000 | 4 |
| Comparative Example 1 | 5000 | +/- | 16000 | 3 |
| Comparative Example 2 | 5000 | -/- | 20 | 44 |

### Example 4

### (1) Preparation of streptavidin-modified substrate

A streptavidin-modified substrate was prepared in the same way as in Example 2 except that streptavidin (manufactured by PIERCE) was used instead of the N-avidin.

The isoelectric point of the streptavidin (manufactured by PIERCE) is approximately pH 7. The streptavidin has a negative surface charge in a solution of a boric acid buffer (pH 8.5).

### (2) Evaluation of biotinylated protein immobilization performance of streptavidin-modified substrate

The streptavidin-modified substrate can immobilize a biotinylated target protein on the substrate surface by use of avidin-biotin interaction in a manner that suppresses denaturation, and has been used as an immobilization method of a target protein.

The sensor surface of the prepared streptavidin-modified substrate was coated with a member made of polypropylene to thereby prepare cells of 1 mm in width (longitudinal), 7.5 mm in length (lateral), and 1 mm in depth. Then, the substrate was placed in a surface plasmon resonance apparatus. An acetic acid buffer was added to the cells. After the substrate was left standing for 20 minutes, a resonance signal was measured. The difference thereof from a resonance signal of a CMD-surface substrate measured in the same way was used as the amount of the streptavidin immobilized. Next, an acetic acid buffer was added to the cells of the streptavidin-modified substrate. After the substrate was left standing for 20 minutes, a biotin-XX-conjugated horseradish-derived peroxidase (manufactured by Molecular Probe; hereinafter, abbreviated to a biotinylated HRP) solution (100 µg/mL, acetic acid buffer) was charged in the cells for 30 minutes. Then, the solution was substituted by an acetic acid buffer. The amount of a change in resonance signal (RU value) at the time of charging the acetic acid buffer before and after charging the biotinylated HRP solution into the cells was used as the amount of the biotinylated HRP immobilized. The results are shown in Table 2.

### Comparative Example 3

A streptavidin-modified substrate was prepared in the same way as in Comparative Example 1 except that streptavidin was used instead of the N-avidin.

This substrate was evaluated in the same way as in Example 4(2) for the amount of the biotinylated HRP immobilized. The evaluation results are shown in Table 2.

As shown in Table 2, the streptavidin-modified substrate prepared by use of the method of the present invention could immobilize thereon the biotinylated protein in large amounts.

**Table 2**

| | Average thickness of streptavidin solution [µm] | Electric charge of streptavidin/electric charge of substrate | Amount of streptavidin immobilized (average) [RU] | Amount of biotinylated HRP immobilized [RU] |
|---|---|---|---|---|
| Example 4 | 5 | -/- | 12000 | 8200 |
| Comparative Example 3 | 5000 | +/- | 6700 | 3800 |

### Example 5

An N-avidin-modified substrate prepared in the same way as in Example 2 was used to evaluate the amount of the N-avidin immobilized and the amount of biotinylated carbonic anhydrase (hereinafter, abbreviated to biotinylated CA) immobilized in the same way as in Example 4(2) except that the biotinylated CA was used instead of the biotinylated HRP. The results are shown in Table 3.

### Example 6

An N-avidin-modified substrate was prepared and evaluated in the same way as in Example 2 except that the difference between dry-bulb and wet-bulb temperatures in the spin coater was set to 13.5°C.

### Example 7

An N-avidin-modified substrate was prepared and evaluated in the same way as in Example 2 except that the difference between dry-bulb and wet-bulb temperatures in the spin coater was set to 7.0°C.

### Example 8

An N-avidin-modified substrate was prepared and evaluated in the same way as in Example 2 except that the difference between dry-bulb and wet-bulb temperatures in the spin coater was set to 2.5°C.

**Table 3**

| | Difference between dry-bulb and wet-bulb temperatures [°C] | Amount of N-avidin immobilized (average) [RU] | Amount of biotinylated CA immobilized [RU] |
|---|---|---|---|
| Example 6 | 13.5 | 12000 | 4100 |
| Example 5 | 10.0 | 11500 | 3200 |
| Example 7 | 7.0 | 12000 | 2900 |
| Example 8 | 2.5 | 12000 | 1800 |

### Example 9

### (1) Preparation of application solution

1.9 g of poly(3,3,4,4,5,5,6,6,7,7,8,8,8-tridecafluorooctyl acrylate)-poly(benzyl methacrylate) copolymer (monomer mass ratio: 4/6, mass-average molecular weight: 30000; hereinafter, abbreviated to a polymer A) was dissolved in methyl isobutyl ketone, and methyl isobutyl ketone was added thereto to bring the amount of the solution to 100 mL. This polymer A solution was filtered through a 0.45-µm filter to prepare an application solution A. The methyl isobutyl ketone used was treated by dehydration in advance with a molecular sieve 4A 1/16 for 16 hours.

### (2) Preparation of polymer A-coated substrate

A sensor stick in which an Au thin film prepared by the method of Example 1(1) was formed was set in an aluminum container having a closed structure of 30 mm in length × 130 mm in width × 10 mm in depth. This aluminum container was secured onto the inner cup of the spin coater used in Example 1(3) so that the gold-surface substrate had its long axis disposed in the tangential direction of a circular arc at a position 135 mm from the center. Onto this gold-surface substrate, 100 µL of the application solution A was added dropwise by use of a micropipette to coat the overall surface of the gold-surface substrate with the application solution A. The aluminum container was closed and left standing for 30 seconds. Then, the substrate was rotated at 200 rpm for 60 seconds. The substrate was left standing in the closed container for 5 minutes and then taken out of the closed container. The solution was dried overnight at room temperature under normal pressure to obtain a polymer A-coated substrate.

### (3) Preparation of hydrophobic polymer-surface substrate

The polymer A-coated chip thus prepared was dipped for 16 hours in a 1 N NaOH aqueous solution warmed at 60°C and then washed under running pure water to prepare a hydrophobic polymer-surface substrate in which a COOH group was introduced in the polymer A-coated layer surface.

### (4) Preparation and evaluation of N-avidin-modified substrate

An avidin-modified substrate was prepared and evaluated for the amount of the N-avidin immobilized in the same way as in Example 2 except that the hydrophobic polymer-surface substrate obtained in (3) was used instead of the CMD-surface substrate. The amount ofN-avidin immobilized is shown in Table 4.

### Example 10

### (1) Preparation of SAM-surface substrate

A sensor stick prepared by the method of Example 1(1) in which an Au thin film was formed was surface-treated at 25°C for 18 hours by adding dropwise 100 µL of a 1 mM ethanol solution of 7-carboxy-1-heptanethiol (Dojindo Laboratories) so as to bring the solution into contact with the gold film. Then, the sensor stick was washed five times with ethanol, once with an ethanol/water mixed solvent, and five times with water. This sample is designated as an SAM-surface substrate.

### (2) Preparation and evaluation of N-avidin-modified substrate

An avidin-modified substrate was prepared and evaluated for the amount of the N-avidin immobilized in the same way as in Example 2 except that the SAM-surface substrate obtained in (1) was used instead of the CMD-surface substrate. The amount of the N-avidin immobilized is shown in Table 4.

### Comparative Example 4

A hydrophobic polymer-surface substrate prepared by the method described in Example 9(1) to (3) was evaluated for the amount of the N-avidin immobilized by the method described in Comparative Example 2. The amount of the N-avidin immobilized is shown in Table 4.

### Comparative Example 5

An SAM-surface substrate prepared by the method of Example 10 was evaluated for the amount of the N-avidin immobilized by the method described in Comparative Example 2. The amount of the N-avidin immobilized is shown in Table 4.

**Table 4**

| | Electric charge of N-avidin/electric charge of substrate | Amount of N-avidin immobilized (average) [RU] |
|---|---|---|
| Example 9 | -/- | 700 |
| Example 10 | -/- | 800 |
| Comparative Example 4 | -/- | 50 |
| Comparative Example 5 | -/- | 30 |

As shown in Table 4, the method of the present invention could immobilize the physiologically active substance even at pH where the electric charges of the substrate surface and the physiologically active substance repelled each other.

Subsequently, in the present invention, the evaluation of a substrate for a sensor for which the amount of a layer for immobilizing a physiologically active substance immobilized and the proportion of a physiologically active substance are adjusted will be shown below.

### Example 11: Substrate of the present invention

A pellet of ZEONEX (ZEON CORP.) was dissolved at 240°C. This dissolution product was molded into a prism substrate of 8 mm in length × 120 mm in width × 1.5 mm with an injection molding machine. The prism substrate was attached to the substrate holder of a parallel plate-type sputtering apparatus for 6 inch (SH-550, manufactured by ULVAC, Inc.). After a vacuum (base pressure: 1×10⁻³ Pa or less) was drawn, Ar gas was introduced (1 Pa). While the substrate holder was rotated (20 rpm), RF power (0.5 kW) was applied to the substrate holder for approximately 9 minutes to plasma-treat the prism surface (substrate). Next, the Ar gas was stopped, and a vacuum was drawn. Ar gas was introduced again (0.5 Pa). While the substrate holder was rotated (10 to 40 rpm), DC power (0.2 kW) was applied to an 8-inch Cr target for approximately 30 seconds to form a Cr thin film of 2 nm. Next, the Ar gas was stopped, and a vacuum was drawn again. Ar gas was introduced again (0.5 Pa). While the substrate holder was rotated (20 rpm), DC power (1 kW) was applied to an 8-inch Au target for approximately 50 seconds to form an Au thin film of approximately 50 nm.

The substrate thus obtained in which the Au thin film was formed was dipped in a 1 mM aqueous solution of 6-Amino-1-hexanethiol, hydrochloride (manufactured by Dojindo Laboratories) at 40°C for 20 hours and washed five times with ultrapure water. This substrate is designated as an amine-surface substrate.

### (2) Active esterification of CMD (carboxymethyldextran)

After 20 g of 1% by weight of a CMD (manufactured by Meito Sangyo Co., Ltd.; average molecular weight: 1000000, substitution degree: 0.67) solution (carboxyl group amount: 5×10⁻⁴ mol) was dissolved, 20 mL of an aqueous solution containing 1 mM 1-ethyl-2,3-dimethylaminopropyl carbodiimide (manufactured by Dojindo Laboratories) was added thereto. The mixture was stirred at room temperature for 3 minutes.

### (3) Binding reaction of CMD to substrate

Onto the substrate prepared in (1), 1 ml of the active-esterified CMD solution prepared in (2) was added dropwise by use of a micropipette to coat, with the solution, the overall prism surface in which the Au thin film was formed. The substrate was secured onto the rotating plate of a spin coater (Model 408 (Special), manufactured by Nanometric Technology Inc.) so that the longitudinal direction of the substrate corresponded to the tangential direction of a circular arc at a position 135 mm in radius from the rotation center. A thin film of active-esterified carboxymethyldextran was formed by spin coating at 1000 rpm for 45 seconds on the substrate having an amino group. After reaction at room temperature for 15 minutes, the substrate was dipped in a 1 N NaOH aqueous solution for 30 minutes and washed five times with ultrapure water to thereby prepare a CMD-surface substrate.

### (4) Preparation of avidin-modified substrate

To the CMD-surface substrate surface prepared in (3), 1 mL of a 1:1 mixed solution of 1-ethyl-2,3-dimethylaminopropyl carbodiimide (400 mM) and N-hydroxysuccinimide (100 mM, manufactured by Wako Pure Chemical Industries, Ltd.) was added dropwise by use of a micropipette to coat the CMD surface therewith. The substrate was left standing at room temperature for 30 minutes to active-esterify the CMD substrate surface. Subsequently, the substrate surface was washed with pure water. The pure water was removed with a nitrogen gun.

The active-esterified CMD-surface substrate was secured on the rotating plate of a spin coater (manufactured by Active Corp.) installed in an environmental testing machine (manufactured by Tokyo-Thermo-Ter Co., Ltd.) adjusted to 23°C and 10% RH (the difference between dry-bulb and wet-bulb temperatures was 13.5°C) so that the longitudinal direction of the substrate corresponded to the tangential direction of a circular arc at a position 135 mm in radius from the rotation center. Onto this substrate, 100 µL of a 5 mg/mL solution of streptavidin (manufactured by Wako Pure Chemical Industries, Ltd.) was added dropwise by use of a syringe pump (manufactured by Harvard Apparatus, Inc.). The solution was spread with a spatula made of polypropylene to coat the overall substrate surface therewith. Then, the substrate was left standing for 1 minute and then rotated at 500 rpm for 45 seconds to dry the solution. Coating with a streptavidin solution and rotation were further repeated twice by the same procedure. Then, the substrate was left standing in the same environmental testing machine for 30 minutes.

To this avidin-modified substrate surface, 1 mL of a 2-aminoethanol hydrochloride aqueous solution (1 M, adjusted to pH 8.5 with 1 N sodium hydroxide) was added dropwise by use of a micropipette. The state where the substrate was coated with the solution was maintained at 23°C for 14 minutes. Subsequently, the substrate was dipped three times in a boric acid buffer (10 mM sodium tetraborate decahydrate was adjusted to pH 8.5 with 1 N hydrochloric acid), then dipped for 10 minutes in a 1 N sodium hydroxide aqueous solution, and subsequently dipped three times in pure water to wash the substrate. Furthermore, the substrate was secured on the rotating plate of a spin coater installed in an environmental testing machine adjusted to 23°C and 10% RH (the difference between dry-bulb and wet-bulb temperatures was 13.5°C) so that the longitudinal direction of the substrate corresponded to the tangential direction of a circular arc at a position 135 mm in radius from the rotation center. The substrate was rotated at 1000 rpm for 45 seconds to prepare a dried avidin-modified surface.

### (5) Measurement of amounts af CMD and avidin immobilized

The prism substrate, and the Au-surface substrate, the amine-surface substrate, the CMD-surface substrate, and the avidin-modified substrate prepared in (1), (3), and (4) were separately measured with a spectroscopic ellipsometer (manufactured by Five Lab Co., Ltd.) to determine the respective optical constants of the ZEONEX, gold, 6-amino-1-hexanethiol layer, CMD layer, streptavidin-immobilized CMD layer. This optical constant was used to calculate the film thicknesses of the immobilized CMD and streptavidin with a monochromatic ellipsometer (manufactured by Five Lab Co., Ltd.). In measurement procedures, first, the CMD film thickness was measured at 10-mm spacings in the longitudinal direction in the central portion of the short axis on the CMD-surface substrate of (3). Subsequently, the film thickness at the same positions of the same substrate after the avidin modification treatment of (4) was measured by use of an ellipsometer. The difference between the avidin-modified CMD film thickness and the CMD film thickness before modification was used as the thickness of the immobilized avidin.

### (6) Measurement of amount of biotinylated protein immobilized on avidin-modified surface

The avidin-modified substrate can immobilize a biotinylated target protein on the substrate surface by use of avidin-biotin interaction in a manner that suppresses denaturation, and has been used as an immobilization method of a target protein. The avidin-modified sensor surface prepared in (4) was coated with a member made of polypropylene to thereby prepare cells of 1 mm in width (longitudinal), 7.5 mm in length (lateral), and 1 mm in depth. One of the avidin-modified substrates thus prepared was evaluated immediately thereafter for a biotin-XX-conjugated horseradish-derived peroxidase (manufactured by Molecular Probe; hereinafter, abbreviated to a biotinylated HRP) binding performance. Another avidin-modified substrate was encapsulated in nitrogen and stored at 45°C for 7 days. Then, the substrate was evaluated for biotinylated HRP binding performance.

To evaluate biotinylated HRP binding performance, the substrate was placed in a surface plasmon resonance apparatus. A PBS buffer (pH 7.4, manufactured by Nippon Gene Co., Ltd.) was added to the cells. After the substrate was left standing for 20 minutes, a biotinylated HRP solution (100 µg/mL, PBS buffer) was charged in the cells for 30 minutes. Then, the solution was substituted by a PBS buffer. The amount of a change in resonance signal (RU value) at the time of charging the PBS buffer before and after charging the biotinylated HRP solution into the cells was used as the amount of the biotinylated HRP immobilized. Storage stability was evaluated as follows: after the avidin-modified substrate was stored in nitrogen at 45°C for 7 days, the amount of the biotinylated HRP immobilized was measured by the method described above. The immobilized amount after storage with respect to that before storage was measured. The ratio between them was evaluated as storage stability (residual rate of immobilization performance). The closer the value is to 1.0, the more excellent the storage stability is. The results are shown in Table 5.

### Example 12:

An avidin-modified substrate was prepared and evaluated for performance by the same procedures as in Example 11 except that 10 mM 1-ethyl-2,3-dimethylaminopropyl carbodiimide was used instead of 1 mM 1-ethyl-2,3-dimethylaminopropyl carbodiimide in (2) Active esterification of CMD (carboxymethyldextran) of Example 11.

### Comparative Example 11:

An avidin-modified substrate was prepared and evaluated for performance by the same procedures as in Example 11 except that the application of streptavidin by spin coating was performed once in (4) Preparation of avidin-modified substrate of Example 11.

### Comparative Example 12:

An avidin-modified substrate was prepared and evaluated for performance by the same procedures as in Example 11 except that the application of streptavidin by spin coating was performed five times in total in (4) Preparation of avidin-modified substrate of Example 11.

### Comparative Example 13:

An avidin-modified substrate was prepared and evaluated for performance by the same procedures as in Example 11 except that 0.05 mM 1-ethyl-2,3-dimethylaminopropyl carbodiimide was used instead of 1 mM 1-ethyl-2,3-dimethylaminopropyl carbodiimide in (2) Active esterification of CMD of Example 11.

**Table 5**

| Substrate | Amount of CMD immobilized [nm] | Amount of avidin immobilized [nm] | Avidin/CMD ratio | Biotinylated HRP immobilization performance | | Remarks |
|---|---|---|---|---|---|---|
| | | | | Immediately after [RU] | Residual ratio of immobilization performance | |
| Example 11 | 10 | 12 | 1.2 | 8000 | 0.8 | The present invention |
| Example 12 | 20 | 20 | 1.0 | 7000 | 0.7 | The present invention |
| Comparative Example 11 | 10 | 2 | 0.2 | 1000 | 0.9 | Comparative Example |
| Comparative Example 12 | 10 | 25 | 2.5 | 6000 | 0.3 | Comparative Example |
| Comparative Example 13 | 2.3 | 4.5 | 2.0 | 1000 | 0.7 | Comparative Example |

As shown in Table 5, the substrate of the present invention immobilized the biotinylated target protein in large amounts on the substrate surface and was also excellent in storage stability. On the other hand, when the ratio of the amount of the avidin immobilized to the amount of the CMD immobilized was low, storage stability was excellent. However, biotinylated target protein binding performance was low, and performance as a sensor surface was reduced. When the ratio was high, binding performance was enhanced. However, storage stability was reduced. When the amount of the CMD immobilized was low, the amount of the avidin immobilized was not increased, and binding performance was low.

### Example 13:

An avidin-modified surface was prepared by the method described in Example 11 except that after coating with a streptavidin solution and rotation were repeated three times, the substrate was left standing for 30 minutes in an environment of 23°C and 50% RH (the difference between dry-bulb and wet-bulb temperatures was 6.7°C). The amount of the biotinylated protein immobilized on this surface was measured by the method described in Example 11. The results are shown in Table 6.

### Example 14:

To a CMD substrate actively-esterified by the method of Example 11, 100 µL of a streptavidin solution was added dropwise by use of a syringe pump by the method of Example 11 in an environmental testing machine adjusted to 23°C and 50% RH (the difference between dry-bulb and wet-bulb temperatures was 6.7°C). The solution was spread with a spatula made of polypropylene to coat the overall substrate surface therewith. Then, the substrate was transferred onto the spin coater described in Example 11. One minute after the overall substrate surface was coated with a streptavidin solution under an environment of 23°C and 10% RH (the difference between dry-bulb and wet-bulb temperatures was 13.5°C), the substrate was rotated at 500 rpm for 45 seconds to dry the solution. An avidin-modified substrate was prepared by the method of Example 11 except that coating with a streptavidin solution at 23°C and 50% RH (the difference between dry-bulb and wet-bulb temperatures was 6.7°C) and drying by spin coating at 23°C and 10% RH (the difference between dry-bulb and wet-bulb temperatures was 13.5°C) were further repeated twice by the same procedure, and then, the substrate was left standing at 23°C and 10% RH (the difference between dry-bulb and wet-bulb temperatures was 13.5°C) for 30 minutes. The amount of the biotinylated protein immobilized on this surface was measured by the method described in Example 11. The results are shown in Table 6.

**Table 6**

| | Difference between dry-bulb and wet-bulb temperatures [°C] | | | Biotinylated HRP immobilization performance [RU] |
|---|---|---|---|---|
| | At the time of avidin solution casting | At the time of drying by spin coating | At the time of reaction after drying | |
| Example 11 | 13.5°C | 13.5°C | 13.5°C | 8000 |
| Example 13 | 13.5°C | 13.5°C | 6.7°C | 6300 |
| Example 14 | 6.7°C | 13.5°C | 13.5°C | 7500 |

As shown in Table 6, the biosensor of the present invention immobilized the biotinylated target protein in large amounts on the substrate surface by making the difference between wet-bulb and dry-bulb temperatures large at the step of coating the substrate surface with a solution containing a physiologically active substance and at the step of drying the solution containing a physiologically active substance and then leaving the substrate standing.

## Claims

1. A method for production of a surface plasmon resonance measurement device comprising the steps of applying to a metal substrate having a layer for immobilizing a physiologically active substance a solution containing a physiologically active substance capable of forming a covalent bond with a molecule constituting the layer for immobilizing a physiologically active substance; and then drying the solution which has been applied onto the surface of the metal substrate within 10 minutes, wherein the method results in covalent immobilisation of said physiologically active substance to said substrate.

2. The method of claim 1 wherein the concentration of the solution containing a physiologically active substance is between 0.1 mg/ml and 10 mg/ml.

3. The method of claim 1 wherein the layer for immobilizing a physiologically active substance is made of a hydrophilic polymer, hydrophobic polymer, self-assembled membrane-forming molecule, or combination thereof.

4. The method of claim 3 wherein the hydrophilic polymer is activated to be covalently bound with the physiologically active substance.

5. The method of claim 3 wherein the thickness of the hydrophilic polymer layer is between 1 nm and 300 nm.

6. The method of claim 3 wherein the hydrophilic polymer comprises a dextran derivative, cellulose compound, polyacrylic acid derivative, or polyvinyl alcohol derivative.

7. The method of claim 1 wherein the solution containing a physiologically active substance is applied thereto at an average liquid film thickness of 300 µm or less.

8. The method of claim 1 wherein the solution containing a physiologically active substance is applied thereto at an average liquid film thickness of 20 µm or less.

9. The method of claim 1 wherein the solution containing a physiologically active substance is applied thereto with a spin coater or dispenser.

10. The method of claim 1 wherein the physiologically active substance is a protein.

11. The method of claim 10 wherein the protein is a protein A, protein G, avidins, calmodulin, or antibody.

## Patentansprüche

1. Verfahren zur Erzeugung einer Oberflächenplasmonresonanzmessvorrichtung, umfassend die Auftragung einer Lösung, umfassend eine physiologisch aktive Substanz, die in der Lage ist, eine kovalente Bindung mit einem Molekül zu bilden, das die Schicht zum Immobilisieren einer physiologisch aktiven Substanz ausmacht, auf ein Metallsubstrat mit einer Schicht zum Immobilisieren einer physiologisch aktiven Substanz; und anschließendes Trocknen der Lösung, die auf die Oberfläche des Metallsubstrates aufgetragen ist, innerhalb von 10 Minuten, worin das Verfahren zur kovalenten Immobilisierung der physiologisch aktiven Substanz an dem Substrat resultiert.

2. Verfahren nach Anspruch 1, worin die Konzentration der Lösung, umfassend eine physiologisch aktive Substanz, zwischen 0,1 mg/ml und 10 mg/ml ist.

3. Verfahren nach Anspruch 1, worin die Schicht zum Immobilisieren einer physiologisch aktiven Substanz aus einem hydrophilen Polymer, hydrophoben Polymer, einem Molekül, das eine selbst zusammengebaute Membran bildet, oder einer Kombination daraus gemacht ist.

4. Verfahren nach Anspruch 3, worin das hydrophile Polymer aktiviert ist, um kovalent mit der physiologisch aktiven Substanz gebunden zu sein.

5. Verfahren nach Anspruch 3, worin die Dicke der hydrophilen Polymerschicht zwischen 1 nm und 300 nm ist.

6. Verfahren nach Anspruch 3, worin das hydrophile Polymer ein Dextranderivat, Celluloseverbindung, Polyacrylsäurederivat oder Polyvinylalkoholderivat umfasst.

7. Verfahren nach Anspruch 1, worin die Lösung, umfassend eine physiologisch aktive Substanz, darauf bei einer durchschnittlichen Flüssigfilmdicke von 300 µm oder weniger aufgetragen wird.

8. Verfahren nach Anspruch 1, worin die Lösung, umfassend eine physiologisch aktive Substanz, darauf bei einer durchschnittlichen Flüssigfilmdicke von 20 µm oder weniger aufgetragen wird.

9. Verfahren nach Anspruch 1, worin die Lösung, umfassend eine physiologisch aktive Substanz, darauf mit einem Spinnbeschichter oder Dispenser aufgetragen wird.

10. Verfahren nach Anspruch 1, worin die physiologisch aktive Substanz ein Protein ist.

11. Verfahren nach Anspruch 10, worin das Protein ein Protein A, Protein G, Avidine, Calmodulin oder Antikörper ist.

## Revendications

1. Procédé de production d'un dispositif de mesure de résonance plasmonique de surface comprenant les étapes d'application à un substrat en métal comportant une couche pour immobiliser une substance physiologiquement active d'une solution contenant une substance physiologiquement active capable de former une liaison covalente avec une molécule constituant la couche pour immobiliser la substance physiologiquement active ; puis de séchage de la solution qui a été appliquée sur la surface du substrat en métal dans l'intervalle de 10 minutes, où le procédé conduit à une immobilisation covalente de ladite substance physiologiquement active sur ledit substrat.

2. Procédé selon la revendication 1, dans lequel la concentration de la solution contenant une substance physiologiquement active est comprise entre 0,1 mg/mL et 10 mg/mL.

3. Procédé selon la revendication 1, dans lequel la couche pour immobiliser une substance physiologiquement active est constituée d'un polymère hydrophile, d'un polymère hydrophobe, d'une molécule formant membrane auto-assemblée, ou de l'une de leurs combinaisons.

4. Procédé selon la revendication 3, dans lequel le polymère hydrophile est activé pour être lié de façon covalente avec la substance physiologiquement active.

5. Procédé selon la revendication 3, dans lequel l'épaisseur de la couche de polymère hydrophile est comprise entre 1 mm et 300 mm.

6. Procédé selon la revendication 3, dans lequel le polymère hydrophile comprend un dérivé de dextrane, un composé de cellulose, un dérivé de poly(acide acrylique) et un dérivé de poly(alcool vinylique).

7. Procédé selon la revendication 1, dans lequel la solution contenant une substance physiologiquement active lui est appliquée à une épaisseur moyenne de film liquide de 300 µm ou moins.

8. Procédé selon la revendication 1, dans lequel la solution contenant une substance physiologiquement active lui est appliquée à une épaisseur moyenne de film liquide de 20 µm ou moins.

9. Procédé selon la revendication 1, dans lequel la solution contenant une substance physiologiquement active lui est appliquée avec une coucheuse centrifuge ou un distributeur.

10. Procédé selon la revendication 1, dans lequel la substance physiologiquement active est une protéine.

11. Procédé selon la revendication 10, dans lequel la protéine est une protéine A, une protéine G, les avidines, la calmoduline ou un anticorps.
